# FASCICULE DE BREVET EUROPEEN

(11) **EP 1 720 441 B1**
(45) Date de publication et mention de la délivrance du brevet: **29.04.2009**
(21) Numéro de dépôt: 05706546.8
(22) Date de dépôt: 03.03.2005
(51) Int. Cl.: A61B 1/015, A61B 1/12

(54) **CONNECTEUR A DETECTEUR DE PRESSION DESTINE A UN SYSTEME D'ENDOSCOPIE**
DRUCKÜBERMITTELNDER VERBINDER FÜR EIN ENDOSKOPIESYSTEM
PRESSURE TRANSMITTING CONNECTOR FOR AN ENDOSCOPY SYSTEM

(30) Priorité: 04.03.2004 FR 0402238
(43) Date de publication de la demande: 15.11.2006
(73) Titulaire: Future Medical System S.A., 1217 Meyrin (CH)
(72) Inventeur: FRANCISCO, André, F-06560 Sophia Antipolis (FR); JANIN, Patrick, F-06000 Nice (FR); PASCAL, Thierry, F-06700 Saint Laurent du Var (FR); DIAS, Armando, F-06300 Nice (FR)
(74) Mandataire: Savoye, Jean-Paul
(86) Numéro de dépôt international: PCT/CH2005/000127
(87) Numéro de publication internationale: WO 2005/084524

(56) Documents cités:
- US-A- 5 044 203
- US-A- 5 643 203

## Description

### Domaine technique

L'invention se rapporte à un connecteur à détection de pression destiné plus particulièrement à un système d'endoscopie, comprenant une voie de communication à fluide, un compartiment borgne ouvert sur la voie de communication par un conduit et fermé par une membrane se déformant en fonction d'une pression dans la voie de communication et un moyen pour transmettre la déformation de la membrane sous la forme d'une grandeur représentative de la pression dans la voie de communication.

### Etat de la technique

Un système d'endoscopie comprenant plus particulièrement une canule pour loger un endoscope et pour former, entre la canule et l'endoscope, un canal d'irrigation ou d'aspiration est décrit notamment par les documents US 5037386 et US 6086542. Le système comprend également une bague de raccordement montée autour de la canule et pourvue d'une voie de raccordement pour communiquer avec le canal d'irrigation ou d'aspiration. II est utilisé dans l'arthroscopie des articulations et plus particulièrement dans l'arthroscopie du genou. L'endoscope est relié à un écran vidéo pour visualiser l'articulation. Le canal d'irrigation ou d'aspiration permet de créer une circulation d'eau physiologique pour maintenir un milieu optiquement clair devant l'endoscope et pour laver l'articulation. La circulation est assurée par une pompe reliée à un réservoir et débitant dans une tubulure raccordée au canal d'irrigation ou d'aspiration par l'intermédiaire de la bague de raccordement.

La pression de l'eau physiologique dans l'articulation est contrôlée par un détecteur de pression à membrane disposé sur une tubulure raccordée par la bague de raccordement à un canal formé dans la canule et dédié à la détection de pression.

Cet agencement présente l'inconvénient de conduire à une détermination erronée de la pression dans certaines conditions d'utilisation, par exemple lorsque la tubulure forme accidentellement un coude entre le détecteur de pression et la bague de raccordement.

Le document US 5044203 divulgue un détecteur de pression à membrane à raccorder sur une telle tubulure. Le détecteur comprend une voie de communication rigide, pourvue d'une ouverture pour communiquer avec un compartiment borgne en forme de tube flexible rapporté autour de la voie de communication. Deux chambres remplies d'huile sont disposées autour du compartiment borgne de façon telle que deux membranes fixées aux chambres à transmission sont mises en contact avec deux parties du compartiment borgne constituant deux autres membranes. Un fluide circulant dans la voie de communication pénètre dans le compartiment borgne par l'ouverture et déforme les membranes du compartiment borgne et des deux chambres de transmission. Une pression d'huile fonction de la déformation des membranes est transmise à des capteurs montés sur chaque chambre à pression d'huile pour une détermination de la pression dans la voie de communication.

Cet agencement présente l'inconvénient selon lequel le compartiment borgne en forme de tube flexible peut être lui-même déformé sur ses flancs et ainsi introduire une cause d'erreur dans la détermination de la pression par rapport à ce qu'elle serait si la déformation des membranes ne résultait que du seul effet de la pression dans la voie de communication.

Le document US 5643203 divulgue un système d'endoscopie du type rappelé précédemment, dans lequel un connecteur est monté sur la bague de raccordement et comprend une voie de communication avec le canal d'irrigation et un détecteur de pression pour détecter une pression dans la voie de communication. La pression de l'eau physiologique dans l'articulation est extrapolée par une loi à partir de la pression détectée dans la voie de communication.

L'extrapolation de la pression dans l'articulation à partir de la pression détectée dans la voie de communication du connecteur permet de s'affranchir d'un canal dédié dans la canule à la prise de pression. On peut ainsi diminuer le diamètre de la canule, dans le but de réduire le traumatisme lors de son introduction dans l'articulation. Par rapport à une tubulure, le connecteur élimine aussi le risque d'une variation de section de la voie de communication et permet une détection fiable de la pression dans cette voie de communication.

Le connecteur est une pièce rigide qui comprend une voie de communication à fluide et un conduit perpendiculaire à la voie de communication. Un détecteur de pression est rapporté sur le connecteur. II comprend un compartiment borgne prévu pour être disposé en regard du conduit pour s'ouvrir dans la voie de communication. Une membrane disposée dans le compartiment borgne est reliée à un transducteur piézoélectrique pour convertir une déformation due à la pression dans la voie de communication en une tension électrique.

Cet agencement présente l'inconvénient selon lequel lors de l'assemblage, il est nécessaire d'agencer avec précision le conduit formé dans le connecteur et le compartiment borgne formé dans le détecteur à transducteur piézoélectrique.

Le but de l'invention est de modifier un connecteur connu dans l'état de la technique illustré précédemment pour en simplifier l'assemblage.

### Divulgation de l'invention

A cet effet, l'invention a pour objet un connecteur à détection de pression destiné plus particulièrement à un système d'endoscopie, comprenant une voie de communication à fluide, un compartiment borgne ouvert sur la voie de communication par un conduit et fermé par une membrane se déformant en fonction d'une pression dans la voie de communication et un moyen pour transmettre la déformation de la membrane sous la forme d'une grandeur représentative de la pression dans la voie de communication, caractérisé en ce que la voie de communication, le conduit et le compartiment borgne sont formés dans une même pièce rigide sur laquelle la membrane est rapportée.

Le report de la membrane sur le compartiment borgne pour refermer ce dernier simplifie l'assemblage du connecteur selon l'invention. De surcroît, la pièce rigide dans laquelle la voie de communication, le conduit et le compartiment borgne sont formés est facilement nettoyée avant le report de la membrane. Enfin, le connecteur tout assemblé est facilement stérilisé.

Dans un mode préféré d'exécution de l'invention, la membrane ferme à la fois le compartiment borgne et une chambre à transmission de pression, de préférence remplie d'air et rapportée sur la pièce rigide pour transmettre la déformation de la membrane sous la forme d'une une pression d'air. Par rapport à un transducteur piézoélectrique, cet agencement élimine le risque d'une détérioration lors d'une opération de stérilisation du connecteur. Par rapport à une transmission par huile, cet agencement élimine également le risque d'une contamination du compartiment borgne et de la voie de communication du connecteur ainsi que du canal d'irrigation ou d'aspiration.

### Brève description des dessins

D'autres avantages de l'invention apparaîtront à la lecture de la description d'un mode de réalisation illustré ci-après par les dessins.

- La figure 1: montre un système d'endoscopie en vue de face.
- La figure 2: montre le système d'endoscopie de la figure 1 en vue de dessus.
- La figure 3: montre le système d'endoscopie de la figure 1 en coupe longitudinale.
- La figure 4: montre le système d'endoscopie de la figure 1 suivant une coupe transversale.
- La figure 5: montre en perspective un connecteur selon l'invention en position pour être connecté à une bague de raccordement d'un système d'endoscopie selon les figures 1 à 4.
- La figure 6: montre en coupe transversale un premier connecteur destiné à un système d'endoscopie selon l'invention.
- La figure 7: montre en coupe transversale un deuxième connecteur destiné à un système d'endoscopie selon l'invention.

### Mode(s) de réalisation de l'invention et application industrielle

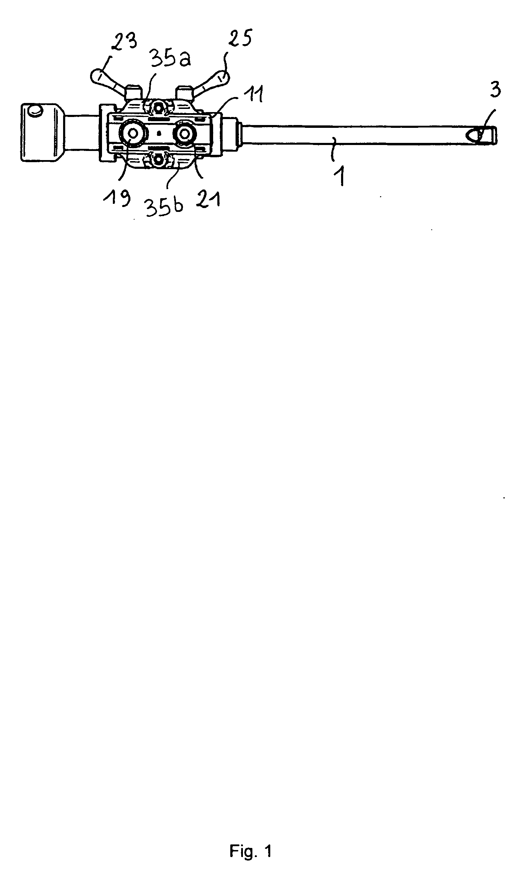

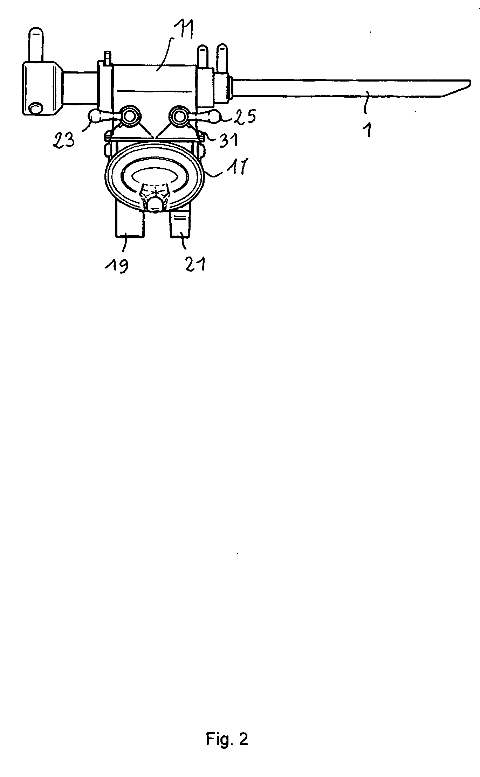

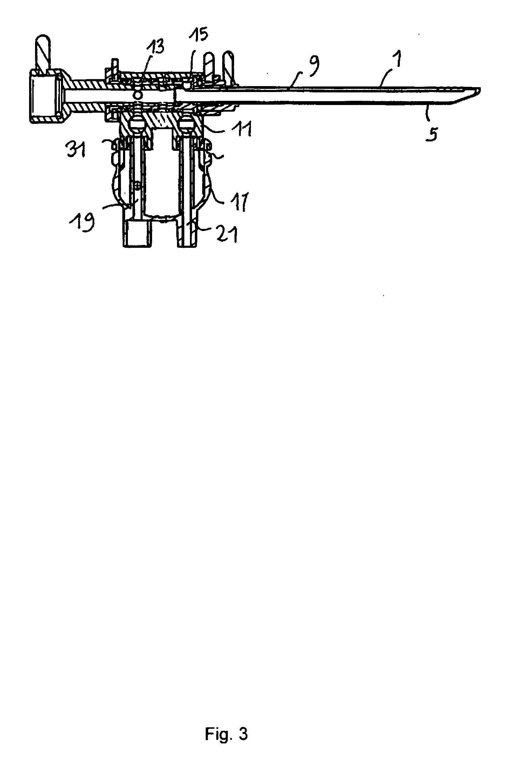

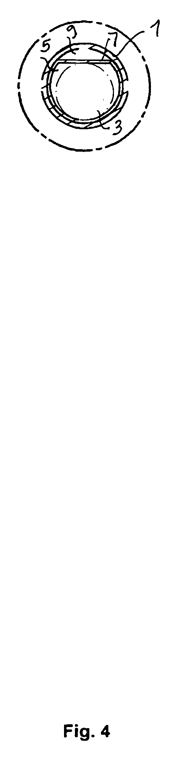

Un système d'endoscopie comprend, figures 1 à 4, une canule 1 pour loger un endoscope 3 et pour former, entre la canule 1 et l'endoscope 3, un canal d'irrigation 5. Dans le mode d'exécution illustré par les figures, le canal d'irrigation 5 est formé entre l'endoscope 3 et un tube 7 interne à la canule 1 et un canal d'aspiration 9 est formé entre le tube interne 7 et la canule 1.

Une bague de raccordement 11 est montée autour de la canule 1 pour communiquer avec le canal d'irrigation 5 et le canal d'aspiration 9. Une première voie de raccordement 13 communique avec le canal d'irrigation 5. Une deuxième voie de raccordement 15 communique avec le canal d'aspiration 9.

Un connecteur 17 est monté sur la bague de raccordement 11. II comprend une première voie de communication 19 pour communiquer avec la première voie de raccordement 13 au canal d'irrigation 5 et une deuxième voie de communication 21 pour communiquer avec la deuxième voie de raccordement 15 au canal d'aspiration 9. Un compartiment borgne 39a,39b est ouvert sur chaque voie de communication 19,21 par un conduit 41a,41b. Une chambre de transmission 35a,35b pourvue d'une membrane 37a,37b est prévue pour qu'une pression dans la voie de communication 19,21 se transmette à la chambre de transmission 35a,35b par déformation de la membrane.

Des tubulures non représentées sont connectées aux voies de communication 19 et 21 du connecteur 17 et reliées à une pompe pour créer une circulation d'eau physiologique propre dans le canal d'irrigation 5 et d'eau physiologique souillée dans canal d'aspiration 9. De façon connue en soi, la bague de raccordement 11 comprend des robinets 23 et 25 pour ouvrir ou fermer les voies de raccordement 13 et 15 en fonction de la circulation recherchée dans le canal d'irrigation 5 ou dans le canal d'aspiration 9.

Le connecteur 17 est monté sur la bague de raccordement 11 pour permettre au canal d'irrigation 5 et au canal d'aspiration 9 de communiquer avec les voies de communication 13 et 15 du connecteur sans l'intermédiaire d'une tubulure. Par cet agencement, la pression détectée sur l'une ou l'autre voie de communication du connecteur n'est pas sujette à une erreur due à une variation accidentelle de la section des tubulures qui seraient raccordées aux voies de raccordement de la bague de raccordement.

La voie de communication 19,21 ainsi que le conduit 41 a,41 b et le compartiment borgne 39a,39b sont formés dans une même pièce rigide 43 sur laquelle la membrane 37a,37b et la chambre de transmission sont rapportées pour fermer la chambre de transmission sur le compartiment borgne 39a,39b par la membrane 37a,37b.

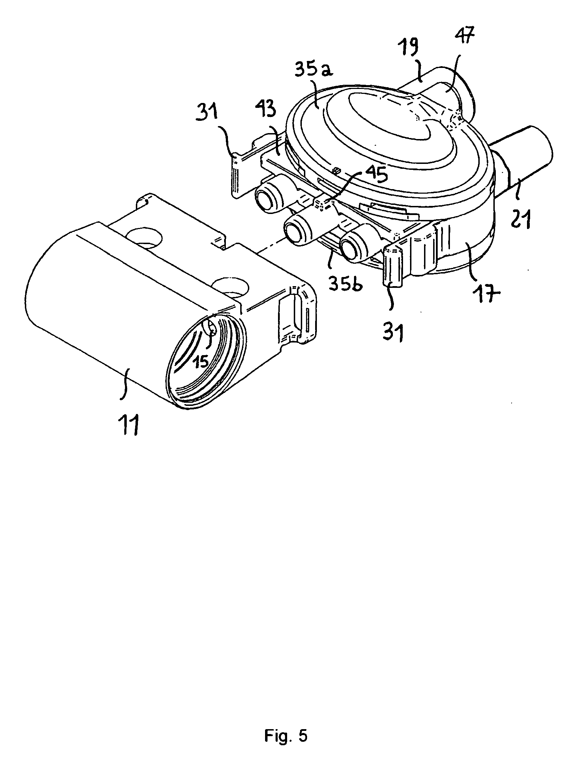

La pièce rigide 43 est pourvue de moyens de fixation 31 pour connecter de façon réversible, figure 5, le connecteur 17 à la bague de raccordement 11. De préférence, la pièce rigide 43 est pourvue d'un organe 45 à détromper la connexion sur la bague de raccordement 11. Ces agencements permettent à un chirurgien de connecter le connecteur sur la bague de raccordement d'une façon aisée et sûre.

L'eau physiologique circulant dans la voie de communication 19 avec le canal d'irrigation 5, ou dans la voie de communication 21 avec le canal d'aspiration 21, pénètre dans le compartiment borgne 39a,39b fermé par la membrane 37a,37b. Cette dernière 37a,37b se déforme en fonction de la pression de l'eau physiologique dans la voie de communication 19 ou 21. Cette déformation engendre une variation de la pression d'air régnant dans la chambre de transmission 35a,35b. Des capillaires non représentés sont branchés sur des prises de branchement 47 des chambres de transmission 37a,37b pour transmettre la variation de pression à des capteurs non représentés et déterminer la pression de l'eau physiologique dans chacune des voies de communication 19, 21.

La pression de l'eau physiologique dans l'articulation est extrapolée par une loi à partir de la pression détectée dans la voie de communication. On utilise de préférence une relation entre le débit de fluide, par donné par la vitesse de rotation de la pompe d'irrigation, ou d'aspiration, et une perte de charge, déterminée expérimentalement, entre la voie de communication du connecteur et l'embouchure du canal d'irrigation ou d'aspiration.

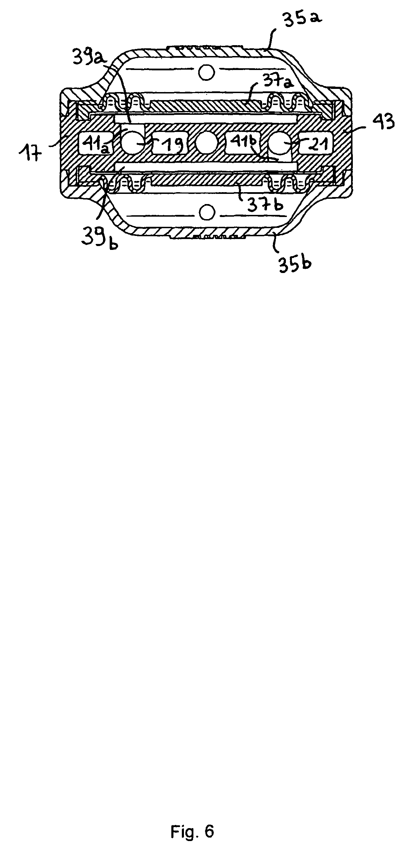

Figure 6, chaque compartiment borgne 39a,39b s'ouvre respectivement sur chaque voie de communication 19,21. Cet agencement permet de détecter de façon indépendante la pression dans chaque voie de communication 19,21. Dans ce mode d'exécution de l'invention, le connecteur 17 permet, dans le système d'endoscopie décrit précédemment, une double détermination de la pression de l'eau physiologique dans l'articulation, par extrapolation à partir de la pression détectée dans la voie de communication 19 avec le canal d'irrigation 5 d'une part et dans la voie de communication 21 avec le canal d'aspiration 9 d'autre part. Avantageusement, la pression dans l'articulation peut être extrapolée par le biais de l'une 19 des deux voies de communication même lorsque la circulation de l'eau physiologique est interrompue dans l'autre 21 voie de communication par la fermeture du robinet d'irrigation 23, respectivement d'aspiration 25. Dans cet agencement également, le chirurgien connecte avantageusement à la bague de raccordement, en une seule opération, la voie de communication pour l'irrigation et la voie de communication pour l'aspiration tout en permettant une détection de pression dans chacune de ces deux voies.

Les deux prises de pression sur les deux voies de communication du connecteur permettent de mieux contrôler l'intégrité du système d'endoscopie décrit précédemment en comparant les pressions détectées avec des valeurs attendues obtenues expérimentalement. En cas de différence, on pourra diagnostiquer une défaillance du robinet d'irrigation 23, du robinet d'aspiration 25, ou diagnostiquer la présence d'un corps étranger dans le canal d'irrigation ou dans le canal d'aspiration. Ces contrôles seront avantageusement effectués par le chirurgien en début d'utilisation du système d'endoscopie.

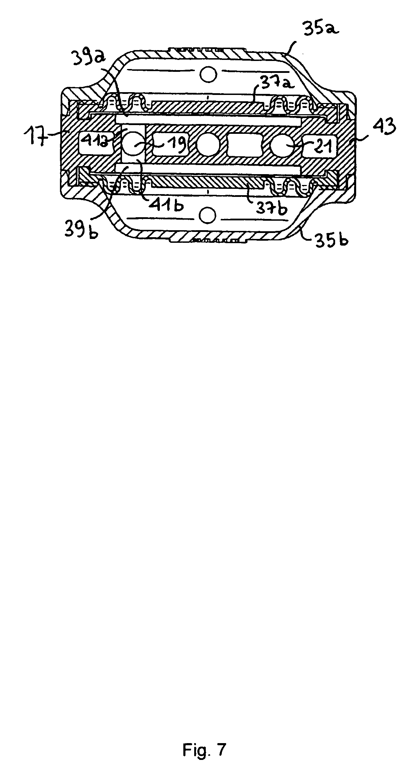

Figure 7, les deux compartiments borgnes 39a,39b s'ouvrent sur la même voie de communication, par exemple 19. Cet agencement permet de dédoubler la détection de la pression dans la voie de communication 19.

De préférence, la pièce rigide 43 est fabriquée par injection de matière plastique. Ce mode de fabrication est avantageux pour obtenir des connecteurs à usage unique.

## Revendications

1. Connecteur à détection de pression destiné plus particulièrement à un système d'endoscopie, comprenant une voie de communication à fluide (19,21), un compartiment borgne (39a,39b) ouvert sur la voie de communication (19,21) par un conduit (41a,41b) et fermé par une membrane (37a,37b) se déformant en fonction d'une pression dans la voie de communication (19,21) et un moyen pour transmettre une grandeur représentative de la pression dans la voie de communication en fonction de la déformation de la membrane, **caractérisé en ce que** la voie de communication (19,21), le conduit (41a,41b) et le compartiment borgne (39a,39b) sont formés dans une même pièce rigide (43) sur laquelle la membrane (37a,37b) est rapportée.

2. Connecteur selon la revendication 1, **caractérisé en ce que** dans la pièce rigide (43) sont formés deux voies de communication (19,21) et deux compartiments borgnes (39a,39b) ouverts chacun sur une des deux voies de communication (19,21) et fermés chacun par une membrane (37a,37c) rapportée sur la pièce rigide (43).

3. Connecteur selon la revendication 2, **caractérisé en ce que** chaque compartiment borgne (39a,39b) s'ouvre respectivement sur chaque voie de communication (19,21).

4. Connecteur selon la revendication 2, **caractérisé en ce que** les deux compartiments borgnes (39a,39b) s'ouvrent sur la même voie de communication (19).

5. Connecteur selon la revendication 1 ou 2, **caractérisé en ce que** la membrane ferme à la fois le compartiment borgne (39a,39b) et une chambre à transmission de pression (35a,35b), rapportée sur la pièce rigide (43) pour convertir la déformation de la membrane (37a,37b) en une pression représentative de la pression dans la voie de communication (19,21).

6. Connecteur selon la revendication 5, **caractérisé en ce que** la chambre à transmission de pression (35a,35b) est remplie d'air pour convertir la déformation de la membrane (37a,37b) en une pression d'air.

7. Connecteur selon la revendication 1 ou 2, **caractérisé en ce que** la pièce rigide (43) est pourvue d'un organe (45) à détromper.

8. Connecteur selon la revendication 1 ou 2, **caractérisé en ce que** la pièce rigide (43) est en matière plastique injectée.

## Claims

1. A pressure-sensing connector intended more particularly for an endoscopy system, comprising a fluid communication path (19, 21), a blind compartment (39a, 39b) that opens onto the communication path (19, 21) via a duct (41a, 41b) and is closed off by a membrane (37a, 37b) that deforms according to the pressure in the communication path (19, 21), and a means for transmitting a quantity representative of the pressure in the communication path according to the deformation of the membrane, **characterized in that** the communication path (19, 21), the duct (41a, 41b) and the blind compartment (39a, 39b) are formed in the same rigid part (43) to which the membrane (37a, 37b) is attached.

2. The connector as claimed in claim 1, **characterized in that** two communication paths (19, 21) and two blind compartments (39a, 39b) are formed in the rigid part (43), each blind compartment opening onto one of the two communication paths (19, 21) and each being closed off by a membrane (37a, 37c) attached to the rigid part (43).

3. The connector as claimed in claim 2, **characterized in that** each blind compartment (39a, 39b) opens onto each communication path (19, 21), respectively.

4. The connector as claimed in claim 2, **characterized in that** the two blind compartments (39a, 39b) open onto the same communication path (19).

5. The connector as claimed in claim 1 or 2, **characterized in that** the membrane closes off both the blind compartment (39a, 39b) and a pressure-transmitting chamber (35a, 35b), connected to the rigid part (43), in order to convert the deformation of the membrane (37a, 37b) into a pressure representative of the pressure in the communication path (19, 21).

6. The connector as claimed in claim 5, **characterized in that** the pressure-transmitting chamber (35a, 35b) is filled with air in order to convert the deformation of the membrane (37a, 37b) into an air pressure.

7. The connector as claimed in claim 1 or 2, **characterized in that** the rigid part (43) is provided with a polarizing feature (45).

8. The connector as claimed in claim 1 or 2, **characterized in that** the rigid part (43) is made of injection-molded plastic.

## Patentansprüche

1. Insbesondere für ein Endoskopiesystem bestimmter Druckmessstecker, der einen Fluidübertragungskanal (19, 21), einen Sacklochraum (39a, 39b), der zum Fluidübertragungskanal (19, 21) über ein Rohr (41 a, 41 b) geöffnet und über eine Membran (37a, 37b), welche sich in Abhängigkeit vom Druck im Übertragungskanal (19, 21) verformt, geschlossen wird und ein Mittel zur Übertragung einer repräsentativen Größe im Übertragungskanal in Abhängigkeit von der Verformung der Membran umfasst, **dadurch gekennzeichnet, dass** der Übertragungskanal (19, 21), das Rohr (41a, 41b) und der Sacklochraum (39a, 39b) in ein und demselben starren Teil (43) integriert sind, an welches die Membran (37a, 37b) angesetzt ist.

2. Druckmessstecker gemäß Anspruch 1, **dadurch gekennzeichnet, dass** in dem starren Teil (43) zwei Übertragungskanäle (19, 21) und zwei geschlossene Sacklochräume (39a, 39b) integriert sind, welche zu einem der beiden Übertragungskanäle (19, 21) geöffnet und über eine am starren Teil (43) angebrachte Membran (37a, 37c) geschlossen werden.

3. Druckmessstecker gemäß Anspruch 2, **dadurch gekennzeichnet, dass** jeder Sacklochraum (39a, 39b) sich jeweils zu jedem Übertragungskanal (19, 21) öffnet.

4. Druckmessstecker gemäß Anspruch 2, **dadurch gekennzeichnet, dass** die beiden Sacklochräume (39a, 39b) sich zum selben Übertragungskanal (19) öffnen.

5. Druckmessstecker gemäß Anspruch 1 oder 2, **dadurch gekennzeichnet, dass** die Membran gleichzeitig das Sackloch (39a, 39b) und eine Druckübertragungskammer (35a, 35b) schließt, welche auf dem starren Teil (43) angebracht ist, um die Verformung der Membran (37a, 37b) im Übertragungskanal (19, 21) in einen repräsentativen Druck umzuwandeln.

6. Druckmessstecker gemäß Anspruch 5, **dadurch gekennzeichnet, dass** die Druckübertragungskammer (35a, 35b) mit Luft gefüllt ist, um die Verformung der Membran (37a, 37b) in einen Luftdruck umzuwandeln.

7. Druckmessstecker gemäß Anspruch 1 oder 2, **dadurch gekennzeichnet, dass** das starre Teil (43) mit einem Berichtigungselement (45) ausgestattet ist.

8. Druckmessstecker gemäß Anspruch 1 oder 2, **dadurch gekennzeichnet, dass** das starre Teil (43) aus Spritzkunststoff gefertigt ist.
